# EUROPEAN PATENT APPLICATION

(11) **EP 4 488 266 A1**
(43) Date of publication of application: **08.01.2025**
(21) Application number: 23780317.6
(22) Date of filing: 27.03.2023
(51) Int. Cl.: C07D 251/34, C07D 229/00, C08G 18/02, C09D 175/04

(54) **BUTANE DIISOCYANATE DERIVATIVE, METHOD FOR PRODUCING BUTANE DIISOCYANATE DERIVATIVE, POLYISOCYANATE COMPOSITION, POLYURETHANE RESIN FORMING COMPOSITION, COATING COMPOSITION AND COATING FILM**

(30) Priority: 31.03.2022 JP 2022058721
(71) Applicant: Tosoh Corporation, Yamaguchi 746-8501 (JP)
(72) Inventor: WADA, Yuya, Yokkaichi-shi, Mie 510-8540 (JP); NAKASHIMA, Yuji, Yokkaichi-shi, Mie 510-8540 (JP)
(74) Representative: TBK
(86) International application number: PCT/JP2023/012145
(87) International publication number: WO 2023/190307

(57) **Abstract**

A butane diisocyanate derivative having an isocyanurate group and a uretdione group, wherein the molar ratio of the isocyanurate groups to the uretdione groups is 20/80 to 80/20, and wherein the weight average molecular weight based on gel permeation chromatography is 790 g/mol or less.

## Description

### Technical Field

The present disclosure relates to a butane diisocyanate derivative, a method for producing a butane diisocyanate derivative, a polyisocyanate composition, a polyurethane resin forming composition, a coating composition and a coating film.

### Background Art

From the viewpoint of reducing the environmental impact, the development of a polyisocyanate composition that achieves a low viscosity and a quick-drying property is being actively conducted. When the viscosity of the polyisocyanate composition is lowered, it is possible to reduce the amount of an organic solvent used in a coating composition. Due to the polyisocyanate composition having an excellent drying property, the thermal energy required for a curing reaction can be reduced.

Here, Patent Literature 1 discloses a coating composition in which a binder contains, in a predetermined ratio, (a) an isocyanurate group-containing polyisocyanate made from 1,4-diisocyanatobutane (BDI) and having an isocyanate content of 20 to 30 wt% and a monomer BDI content of less than 2 wt%, and (b) a polyol component. According to Patent Literature 1, this isocyanurate group-containing polyisocyanate can be rapidly dried under mild curing conditions to form a coating.

In addition, Patent Literature 2 discloses a polyisocyanate composition having a predetermined viscosity including a copolymerized polyisocyanate formed of aliphatic diisocyanate units and alicyclic diisocyanate units and/or an aliphatic polyisocyanate formed of aliphatic diisocyanate units and an alicyclic polyisocyanate formed of alicyclic diisocyanate units, wherein the molar ratio of the aliphatic diisocyanate units/the alicyclic diisocyanate units in the polyisocyanate composition is a predetermined ratio, and the polyisocyanate composition contains a polyisocyanate having a predetermined content of isocyanurate groups. According to Patent Literature 2, this polyisocyanate composition has a low viscosity and an excellent quick-drying property.

### Citation List

### Patent Literature

[Patent Literature 1] Japanese Unexamined Patent Publication No. H9-132751
[Patent Literature 2] Japanese Unexamined Patent Publication No. 2015-127368

### Summary of Invention

### Technical Problem

However, the isocyanurate group-containing polyisocyanate described in Patent Literature 1 has a problem of the viscosity being increased and the amount of a diluting solvent used being increased. In addition, in the polyisocyanate composition described in Patent Literature 2, the hardness of the obtained coating film has not been considered, and it is insufficient to achieve both a low viscosity and a high hardness.

Here, although there has been a very strong market demand for reducing the environmental impact, particularly in recent years, as described above, it has been difficult to achieve a polyisocyanate composition that has a low viscosity and an excellent quick-drying property and is useful for producing a coating film having a high hardness.

Accordingly, one aspect of the present disclosure is directed to provide a polyisocyanate composition that has a low viscosity and an excellent quick-drying property, and is useful for producing a coating film having a high hardness. Other aspects of the present disclosure are directed to provide a butane diisocyanate derivative that is useful for producing the polyisocyanate composition and a method for producing the butane diisocyanate derivative. Still other aspects of the present disclosure are directed to provide a polyurethane resin forming composition, a coating composition and a coating film, which use the polyisocyanate composition.

### Solution to Problem

In some aspects, the present disclosure provides the following [1] to [11].
[1] A butane diisocyanate derivative having an isocyanurate group and a uretdione group,
   wherein the molar ratio of the isocyanurate groups to the uretdione groups is 20/80 to 80/20, and
   wherein the weight average molecular weight based on gel permeation chromatography is 790 g/mol or less.
[2] The butane diisocyanate derivative according to [1], including a butane diisocyanate trimer and a butane diisocyanate dimer,
   wherein a total content of the butane diisocyanate trimer and the butane diisocyanate dimer based on a total mass of the butane diisocyanate derivatives is 45 to 95 mass%.
[3] The butane diisocyanate derivative according to [1] or [2],
   wherein the molar ratio of the isocyanurate groups to the uretdione groups is 50/50 or more.
[4] The butane diisocyanate derivative according to any one of [1] to [3],
   wherein the molar ratio of the isocyanurate groups to the uretdione groups is 60/40 or less.
[5] A method for producing the butane diisocyanate derivative according to any one of [1] to [4], including:
   a first heating step of heating a reaction product containing butane diisocyanate at 80°C or higher; and
   a second heating step of heating the reaction product that has undergone the first heating step at 80 to 180°C in the presence of an isocyanurate-forming catalyst.
[6] The method for producing a butane diisocyanate derivative according to [5],
   wherein the isocyanurate-forming catalyst contains an aminosilyl group-containing compound.
[7] A polyisocyanate composition including the butane diisocyanate derivative according to any one of [1] to [4].
[8] The polyisocyanate composition according to [7],
   wherein the viscosity at 25°C is 2,500 mPa·s or less.
[9] A polyurethane resin forming composition including the polyisocyanate composition according to [7] or [8] and a polyol.
[10] A coating composition including the polyisocyanate composition according to [8] and a compound having two or more isocyanate-reactive groups.
[11] A coating film including a cured product of the coating composition according to [10].

### Advantageous Effects of Invention

According to one aspect of the present disclosure, it is possible to provide a polyisocyanate composition that has a low viscosity and an excellent quick-drying property, and is useful for producing a coating film having a high hardness. According to other aspects of the present disclosure, it is possible to provide a butane diisocyanate derivative that is useful for producing the polyisocyanate composition and a method for producing the butane diisocyanate derivative. According to still other aspects of the present disclosure, it is possible to provide a polyurethane resin forming composition, a coating composition and a coating film, which use the polyisocyanate composition.

### Description of Embodiments

In this specification, a numerical range indicated by using "to" indicates a range including numerical values stated before and after "to" as a minimum value and a maximum value. In the numerical ranges stated stepwise in this specification, the upper limit value or the lower limit value of the numerical range in a certain stage may be replaced with the upper limit value or the lower limit value of the numerical range in another stage. In addition, in the numerical ranges stated in this specification, the upper limit value or the lower limit value of the numerical range may be replaced with values shown in examples. In addition, the upper limit value and the lower limit value stated individually can be arbitrarily combined.

Hereinafter, preferable embodiments of aspects of the present disclosure will be described. However, the aspects of the present disclosure are not limited to the following embodiments.

### <Butane diisocyanate derivative and polyisocyanate composition>

A butane diisocyanate derivative according to one aspect of the present disclosure has an isocyanurate group and a uretdione group, wherein the molar ratio of the isocyanurate groups to the uretdione groups (a total number of moles of isocyanurate groups/a total number of moles of uretdione groups) is 20/80 to 80/20, and the weight average molecular weight based on gel permeation chromatography is 790 g/mol or less. In the following description, butane diisocyanate may be abbreviated as "BDI," and the molar ratio of the isocyanurate groups to the uretdione groups may be abbreviated as "molar ratio (I/U)."

Using the BDI derivative, it is possible to prepare a polyisocyanate composition that has a low viscosity and an excellent quick-drying property and is useful for producing a coating film having a high hardness. Here, the fact that the polyisocyanate composition has a low viscosity can be confirmed, for example, from the viscosity at 25°C measured with a rheometer. In addition, the fact that the polyisocyanate composition has an excellent quick-drying property can be confirmed, for example, from the curing start temperature of a coating film formed of a coating composition prepared using the polyisocyanate composition. In addition, the fact that the coating film obtained using the polyisocyanate composition has a high hardness can be confirmed, for example, from the Martens hardness of the coating film at 23°C.

A polyisocyanate composition according to another aspect of the present disclosure contains a BDI derivative having an isocyanurate group and a uretdione group, and a molar ratio (I/U) of 20/80 to 80/20. The weight average molecular weight of the BDI derivative based on gel permeation chromatography is preferably 790 g/mol or less. That is, the BDI derivative is preferably the above-described BDI derivative.

The polyisocyanate composition tends to have a low viscosity and an excellent quick-drying property. In addition, a coating film having a high hardness is easily obtained using the polyisocyanate composition.

The BDI derivative is a mixture of a plurality of compounds derived from the BDI monomer. The BDI derivative includes, for example, an isocyanurate form of BDI and a uretdione form of BDI. The isocyanurate form of BDI is a compound having an isocyanurate group (a group formed by cyclopolymerization of three BDI monomer molecules) among compounds derived from BDI monomers. The uretdione form of BDI is a compound having a uretdione group (a group formed by cyclopolymerization of two BDI monomer molecules) among compounds derived from BDI monomers. The BDI derivative may include a compound having an isocyanurate group and a uretdione group (a compound which is both an isocyanurate form and a uretdione form) among compounds derived from BDI monomers. Such a compound corresponds to both an isocyanurate form of BDI and a uretdione form of BDI.

Examples of BDI monomers, which are raw materials for the isocyanurate form of BDI and the uretdione form of BDI include 1,2-butane diisocyanate, 1,3-butane diisocyanate, 1,4-butane diisocyanate, 2,3-butane diisocyanate, and mixtures thereof. Raw materials for the isocyanurate form of BDI and the uretdione form of BDI preferably contain 1,4-butane diisocyanate due to high reactivity of the isocyanate group bonded to the primary carbon. The proportion of 1,4-butane diisocyanate in the raw material for the isocyanurate form of BDI and the proportion of the 1,4-butane diisocyanate in the raw material for the uretdione form of BDI both may be 90 mass% or more, 95 mass% or more or 100 mass%. In the following, an embodiment in which 1,4-butane diisocyanate is used as a BDI monomer will be exemplified, but unless otherwise mentioned, the present disclosure is not limited thereto.

The isocyanurate form of BDI preferably includes an isocyanurate trimer of BDI. The isocyanurate trimer of BDI is, for example, a compound represented by the following Formula (1).

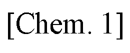

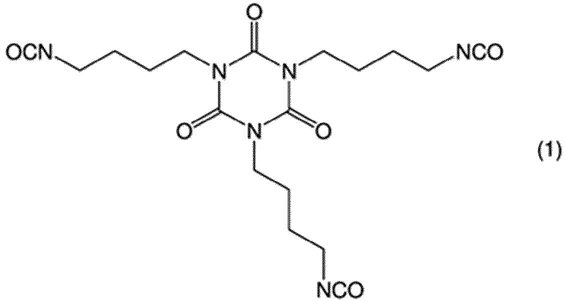

The isocyanurate form of BDI may include a multimer equal to or larger than tetramer formed by multimerization by reacting an isocyanate group (which may be abbreviated hereinafter as "NCO group") in the isocyanurate trimer of BDI with an NCO group in another BDI monomer (or a compound derived from another BDI monomer). The isocyanurate form of BDI more preferably includes a isocyanurate trimer of BDI and the multimer equal to or larger than tetramer, and still more preferably includes an isocyanurate trimer of BDI, an isocyanurate pentamer of BDI (a compound formed by cyclopolymerization of one NCO group in an isocyanurate trimer of BDI with two molecules of another BDI monomer), and an isocyanurate heptamer of BDI (a compound formed by cyclopolymerization of each of two NCO groups in an isocyanurate trimer of BDI with two molecules of another BDI monomer). As described above, the isocyanurate form of BDI may include, as the multimer equal to or larger than tetramer, a compound having a uretdione group (for example, a compound formed by cyclopolymerization of one or two NCO groups in an isocyanurate trimer of BDI with one molecule of another BDI monomer).

The uretdione form of BDI preferably includes an uretdione dimer of BDI. The uretdione dimer of BDI is, for example, a compound represented by the following Formula (2).

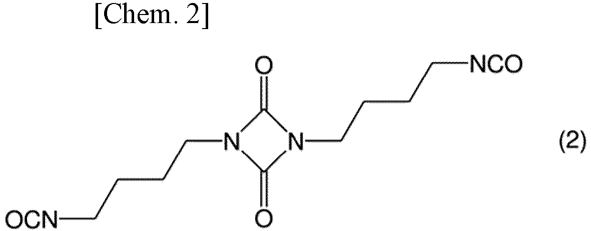

The uretdione form of BDI may include a multimer equal to or larger than trimer formed by multimerization by reacting an NCO group in an uretdione dimer of BDI with an NCO group in another BDI monomer (or a compound derived from another BDI monomer). The uretdione form of BDI more preferably includes an uretdione dimer of BDI and the multimer equal to or larger than trimer, and still more preferably includes an uretdione dimer of BDI, an uretdione trimer of BDI (a compound formed by cyclopolymerization of one NCO group in an uretdione dimer of BDI with one molecule of another BDI monomer), and an uretdione tetramer of BDI (a compound formed by cyclopolymerization of each of two NCO groups in an uretdione dimer of BDI with one molecule of another BDI monomer). As described above, the uretdione form of BDI may include a compound having an isocyanurate group as multimer equal to or larger than trimer (for example, a compound formed by cyclopolymerization of one or two NCO groups in an uretdione dimer of BDI with two molecules of another BDI monomer).

The BDI derivative has a molar ratio (I/U). The molar ratio (I/U) is a molar ratio of isocyanurate groups to uretdione groups in the BDI derivative, and can be rephrased as the molar ratio of isocyanurate groups in the isocyanurate form of BDI and uretdione groups in the uretdione form of BDI. The molar ratio (I/U) of the BDI derivative is 20/80 to 80/20, and may be 25/75 or more, 30/70 or more or 50/50 or more, and may be 75/25 or less, 70/30 or less or 60/40 or less. When the molar ratio (I/U) is 50/50 or more, a coating film with higher hardness tends to be obtained. When the molar ratio (I/U) is 60/40 or less, the viscosity tends to be further reduced. The molar ratio (I/U) can be measured, for example, by a ¹H-NMR method using a nuclear magnetic resonance device (NMR). More specifically, it can be measured according to an example to be described below.

The BDI derivative and the polyisocyanate composition preferably contain a BDI trimer (for example, an isocyanurate trimer of BDI and an uretdione trimer of BDI), and a BDI dimer (for example, a uretdione dimer of BDI). In this case, the viscosity of the polyisocyanate composition becomes lower and the workability tends to be better.

The total content of the BDI trimer and the BDI dimer is preferably 45 mass% or more, more preferably 50 mass% or more, and still more preferably 55 mass% or more, from the viewpoint of promoting a decrease in viscosity of the polyisocyanate composition. The total content of the BDI trimer and the BDI dimer is preferably 95 mass% or less, more preferably 90 mass% or less, and still more preferably 85 mass% or less, from the viewpoint of increasing the crosslink density of the resin. From these viewpoints, the total content of the BDI trimer and the BDI dimer may be, for example, 45 to 95 mass%. The total content of the BDI trimer and the BDI dimer may be 60 mass% or more, 65 mass% or more, 75 mass% or more or 80 mass% or more, and may be 80 mass% or less, 75 mass% or less, 70 mass% or less or 65 mass% or less.

The content of the BDI trimer may be 20 mass% or more, 30 mass% or more, 35 mass% or more or 40 mass% or more, from the viewpoint of promoting an increase in hardness of the coating film. The content of the BDI trimer may be 90 mass% or less, 65 mass% or less, 55 mass% or less or 45 mass% or less, from the viewpoint of promoting a decrease in viscosity of the polyisocyanate composition. From these points, the content of the BDI trimer may be, for example, 20 to 90 mass% or 20 to 65 mass%.

The content of the BDI dimer may be 10 mass% or more, 15 mass% or more, 20 mass% or more or 35 mass% or more, from the viewpoint of promoting a decrease in viscosity of the polyisocyanate composition. The content of the BDI dimer may be 80 mass% or less, 65 mass% or less, 50 mass% or less or 40 mass% or less, from the view point of promoting an increase in hardness of the coating film. From these viewpoints, the content of the BDI dimer may be, for example, 10 to 80 mass% or 10 to 65 mass%.

The above-described total content of the BDI trimer and BDI dimer, and respective contents of the BDI trimer and the BDI dimer are contents based on the total mass of the BDI derivatives or the total mass of the solid content in the polyisocyanate composition, and are values calculated from the differential refractive index peak of the BDI derivative or the polyisocyanate composition based on gel permeation chromatography (GPC). More specifically, these amounts can be measured according to an example to be described below.

The BDI derivative and the polyisocyanate composition may contain a compound derived from a BDI monomer (for example, a biuret form of BDI) in addition to the isocyanurate form of BDI and the uretdione form of BDI, but the content of the compound (hereinafter referred to as "other BDI derivatives") is preferably 20 mol% or less and more preferably 1 mol% or less (substantially free of the compound). The above-described content is a content based on the total number of moles of isocyanurate groups, uretdione groups, and other BDI derivatives (for example, a biuret form of BDI) in the BDI derivative.

The NCO content of the BDI derivative based on the total mass of the solid content in the polyisocyanate composition may be 10 mass% or more or may be 20 mass% or more or 25 mass% or more, from the viewpoint of increasing the number of NCO groups used for crosslinking the polyurethane resin. The NCO content of the BDI derivative based on the total mass of the solid content in the polyisocyanate composition may be 36 mass% or less, 33 mass% or less or 30 mass% or less, from the viewpoint of reducing the content of a low-molecular-weight component such as a BDI monomer to reduce the generation of odor. From these viewpoints, the NCO content of the BDI derivative based on the total mass of the solid content in the polyisocyanate composition may be, for example, 10 to 36 mass%.

The BDI derivative may be blocked with a known blocking agent for the purpose of prolonging the pot life and form the coating composition into one liquid. That is, a part or all of the NCO groups in the isocyanurate form of BDI may be blocked with a blocking agent, and a part or all of the NCO groups in the uretdione form of BDI may be blocked with a blocking agent. The blocked polyisocyanate is inactive at room temperature, but when heated, the blocking agent dissociates, and the isocyanate group becomes activated again, and then reacts with an active hydrogen group. The blocking agent is a compound having one active hydrogen in its molecule, and for example, known blocking agents such as alcohol-based, alkylphenol-based, phenol-based, active methylene, mercaptan-based, acid amide-based, acid imide-based, imidazole-based, urea-based, oxime-based, amine-based, imide-based, and pyrazole-based blocking agents can be used. Here, when the BDI derivative is blocked, the weight average molecular weight and viscosity to be described below are measured after the blocking agent is dissociated.

The weight average molecular weight of the BDI derivative based on gel permeation chromatography (GPC) is 790 g/mol or less, preferably 750 g/mol or less, and more preferably 710 g/mol or less. When the weight average molecular weight of the BDI derivative is within the above-described range, it is possible to obtain a polyisocyanate composition having favorable compatibility with a main agent and a low viscosity. The weight average molecular weight may be 690 g/mol or less, 650 g/mol or less, 600 g/mol or less or 550 g/mol or less. The weight average molecular weight may be 400 g/mol or more, 450 g/mol or more, 500 g/mol or more, 550 g/mol or more or 600 g/mol or more, from the viewpoint of promoting an increase in hardness of the coating film. For the same reason, the weight average molecular weight of the polyisocyanate composition based on gel permeation chromatography may also be within the above-described range. The above-described weight average molecular weight, more specifically, can be measured according to an example to be described below.

The polyisocyanate composition may contain a component other than the BDI derivative, or may be formed of only the BDI derivative. Examples of other components include 1,6-hexane diisocyanate (which may be abbreviated hereinafter as "HDI") and its derivatives.

The polyisocyanate composition may contain, as another component, an isocyanate compound other than the BDI derivative (for example, polyisocyanates such as a BDI monomer, an HDI monomer, and an HDI derivative), but the content of the BDI monomer in the polyisocyanate composition is preferably 5 mass% or less and more preferably 1 mass% or less (substantially free of the BDI monomer). In addition, the content of the isocyanate compound other than the BDI derivative in the polyisocyanate composition is preferably 50 mass% or less and more preferably 1 mass% or less (substantially free of the isocyanate compound other than the BDI derivative). The above-described content is a content based on the total mass of the solid content in the polyisocyanate composition.

The polyisocyanate composition may contain an organic solvent to be described below as another component, but the content of the organic solvent in the polyisocyanate composition is preferably 50 mass% or less and more preferably 20 mass% or less (substantially free of the organic solvent). The above-described content is a content based on the total mass of the solid content in the polyisocyanate composition.

The viscosity of the polyisocyanate composition at 25°C is preferably 3,000 mPa·s or less, more preferably 2,500 mPa·s or less, still more preferably 2,000 mPa·s or less, yet more preferably 1,000 mPa·s or less, and particularly preferably 500 mPa·s or less. When the viscosity is 2,500 mPa·s or less, the polyisocyanate composition has favorable workability without being diluted with a large amount of a solvent, and the solid content concentration of the coating composition can be further increased. The viscosity of the polyisocyanate composition at 25°C may be, for example, 50 mPa·s or more, 100 mPa·s or more or 150 mPa·s or more. When the viscosity is 50 mPa·s or more, the obtained coating film tends to have a higher hardness. The preferable range of the viscosity of the BDI derivative at 25°C is the same as the above-described range in that the polyisocyanate composition having the above-described viscosity can be easily obtained.

The BDI derivative and polyisocyanate composition described above are suitably used as a curing agent component of a coating composition. That is, another embodiment of the present disclosure is a curing agent for coating containing the above-described BDI derivative, and still another embodiment of the present disclosure is a curing agent for coating formed of the above-described polyisocyanate composition. Here, as the main agent for these curing agents for coating, an isocyanate-reactive compound (such as a polyol) to be described below can be used.

### <Method for producing BDI derivative>

Another aspect of the present disclosure is a method for producing the BDI derivative according to the above-described embodiment. The method for producing the BDI derivative includes a multimerization step of obtaining multimers of BDI (an isocyanurate form of BDI and a uretdione form of BDI) by performing an isocyanurate-forming reaction to form an isocyanurate group and a uretdione-forming reaction to form a uretdione group.

The isocyanurate-forming reaction may be performed, for example, in the presence of a generally known isocyanurate-forming catalyst. Examples of isocyanurate-forming catalysts include quaternary onium salts, metal carboxylates, and aminosilyl group-containing compounds. The isocyanurate-forming catalysts may be used alone or two or more thereof may be used in combination.

As the isocyanurate-forming catalyst, from the viewpoint of curbing the generation of a high-molecular-weight component, it is preferable to use an aminosilyl group-containing compound such as aminosilane and silylurea, it is more preferable to use a disilazane such as hexamethyldisilazane and heptamethyldisilazane, and it is still more preferable to use hexamethyldisilazane.

The amount of the isocyanurate-forming catalyst used with respect to a preparation amount of 100 parts by mass of BDI is preferably 0.01 to 10 parts by mass and more preferably 0.1 to 3 parts by mass. When the amount of the isocyanurate-forming catalyst used is larger, the molar ratio (I/U) tends to be larger.

The reaction temperature in the isocyanurate-forming reaction is preferably 80 to 180°C and more preferably 100 to 160°C. When the reaction temperature in the isocyanurate-forming reaction is 80°C or higher, the reaction time can be shortened and thus coloration of the polyisocyanate composition can be further reduced. In addition, when the reaction temperature in the isocyanurate-forming reaction is 80 to 180°C, it is easy to adjust the molar ratio (I/U) to 20/80 to 80/20.

The isocyanurate-forming reaction may be terminated by adding a reaction terminator when a desired isocyanate group content and molecular weight are reached. When the reaction temperature in the isocyanurate-forming reaction is 80 to 180°C, the reaction time of the isocyanurate-forming reaction is, for example, 1 to 5 hours.

The reaction terminator is a compound that has an effect of deactivating the catalyst. As the reaction terminator, for example, water, or a compound having a hydroxy group can be used. The reaction terminator is preferably an alcohol. The reaction terminators may be used alone or two or more thereof may be used in combination. Here, it is preferable to add the reaction terminator quickly after a desired isocyanate group content and molecular weight are reached.

The amount of the reaction terminator added varies depending on the type of the reaction terminator, the type of the isocyanurate-forming catalyst used, or the like, and is preferably 0.1 to 3.5 equivalent, and particularly preferably 0.8 to 2.5 equivalents per equivalent of the isocyanurate-forming catalyst. When the amount of the reaction terminator used is 0.1 equivalents or more per equivalent of the isocyanurate-forming catalyst, the storage stability of the obtained polyisocyanate composition is further improved. When the amount of the reaction terminator used is 3.5 equivalents or less per equivalent of the isocyanurate-forming catalyst, the generation of a reaction product of the reaction terminator and BDI can be curbed, and thus a decrease in the isocyanate group content can be reduced to a greater extent.

The uretdione-forming reaction may be performed, for example, in the presence of a generally known uretdione-forming catalyst. Examples of uretdione-forming catalysts include tertiary phosphines such as trialkylphosphine and tribenzylphosphine, and Lewis acids such as boron trifluoride and zinc trichloride.

When a uretdione-forming catalyst is used, the uretdione-forming reaction may be terminated by adding phosphoric acid, methyl p-toluenesulfonate or the like when the molar ratio (I/U) reaches a desired ratio.

The uretdione-forming reaction can also be performed by heating the reaction product (for example, a BDI monomer) without using a uretdione-forming reaction catalyst as described above. In view of curbing the generation of by-products, it is preferable to perform the uretdione-forming reaction by simply heating without using a uretdione-forming reaction catalyst. The heating temperature is preferably 80°C or higher, more preferably 100 to 170°C, and still more preferably 110 to 160°C. When the heating temperature is 80 to 170°C, it is easy to adjust the molar ratio (I/U) to 20/80 to 80/20 while curbing the generation of by-products.

The isocyanurate-forming reaction and the uretdione-forming reaction may be performed under an inert gas atmosphere such as nitrogen and argon. Specifically, BDI is introduced into a reaction container, an inert gas such as nitrogen or argon is introduced into the container to make the reaction atmosphere an inert gas atmosphere, and the isocyanurate-forming reaction and the uretdione-forming reaction may then be performed by the above-described method.

The isocyanurate-forming reaction and the uretdione-forming reaction may be performed in the absence of an organic solvent or in the presence of an organic solvent. When the reaction is performed in the presence of an organic solvent, it is preferable to use an organic solvent that has no risk of affecting the reaction and a boiling point equal to or higher than the reaction temperature. Examples of organic solvents include aliphatic hydrocarbons such as octane, alicyclic hydrocarbons such as cyclohexane and methylcyclohexane, ketones such as methyl isobutyl ketone and cyclohexanone, esters such as butyl acetate and isobutyl acetate, glycol ether esters such as ethylene glycol ethyl ether acetate, propylene glycol monomethyl ether acetate, 3-methyl-3-methoxybutyl acetate, and ethyl-3-ethoxypropionate, ethers such as dioxane, halogenated hydrocarbons such as methylene iodide and monochlorobenzene, and polar aprotic solvents such as N-methylpyrrolidone, dimethylformamide, dimethylacetamide, dimethylsulfoxide, and hexamethylphosphonylamide. The organic solvents may be used alone or two or more thereof may be used in combination.

The isocyanurate-forming reaction and the uretdione-forming reaction can be performed sequentially or in parallel. When the isocyanurate-forming reaction and the uretdione-forming reaction are performed in parallel, the timings of starting the reaction and terminating the reaction may be simultaneous or different. For example, the uretdione-forming reaction for BDI may be started to proceed the uretdione formation of BDI, and then the uretdione-forming reaction and the isocyanurate-forming reaction may be performed on the reaction product containing a uretdione form of BDI. In the multimerization step, preferably, the isocyanurate-forming reaction and the uretdione-forming reaction are performed simultaneously. In the multimerization step, more preferably, after the uretdione-forming reaction by heat (after the reaction starts), the isocyanurate-forming reaction and the termination reaction are performed. When the uretdione-forming reaction by heat is performed after the isocyanurate-forming reaction is terminated, a side reaction between the catalyst decomposition product by-produced during the termination reaction and isocyanate is promoted, and thickening, a decrease in the isocyanate group content, and the like may occur, and thus it is preferable not to perform the uretdione-forming reaction after the isocyanurate-forming reaction is terminated.

More specifically, the multimerization step is preferably a step including a first heating step of heating a reaction product containing BDI (BDI monomer) at 80°C or higher and a second heating step of heating the reaction product that has undergone the first heating step at 80 to 180°C in the presence of the isocyanurate-forming catalyst. In this method, at least in the first heating step, the uretdione-forming reaction of BDI proceeds, and in the second heating step, the isocyanurate-forming reaction of a reaction product containing a uretdione form of BDI (for example, a mixture of an unreacted BDI monomer and a uretdione form of BDI) proceeds. The heating conditions in the first heating step are the same as the preferable range of the heating temperature in the above-described uretdione-forming reaction, and the heating conditions in the second heating step are the same as the preferable range of the reaction temperature in the above-described isocyanurate-forming reaction. Here, in the second heating step, the uretdione-forming reaction may proceed simultaneously with the isocyanurate-forming reaction.

The first heating step may be a step of heating to 80°C or higher and then holding the reaction product at a temperature of 80°C or higher, from the viewpoint of increasing the amount of the uretdione form. The holding temperature is the same as the preferable range of the heating temperature in the uretdione-forming reaction. The holding time is preferably 1 to 6 hours and more preferably 2 to 5 hours.

After the reaction is completed, the unreacted BDI monomer (free BDI) may be removed from the reaction solution containing the reaction product. That is, the method for producing the BDI derivative may further include a BDI removal step of removing free BDI after the multimerization step. In order to remove free BDI, for example, a removal method by thin-film distillation at 90 to 130°C under a high vacuum of 10 to 100 Pa, an extraction method using an organic solvent or the like is used. The removal is preferably performed so that the residual content of free BDI remaining in the reaction solution is 5 mass% or less. When the residual content of free BDI is 5 mass% or less, it is possible to further reduce the generation of odor, and to further reduce a decrease in storage stability. Here, when an organic solvent is used in the reaction, the organic solvent can be removed simultaneously with the removal of free BDI.

The method for producing the BDI derivative may further include a step of blocking the BDI derivative with the above-described blocking agent after the multimerization step or after the BDI removal step.

The BDI derivative (or a reaction solution containing a BDI derivative) obtained by the method can be directly used as a polyisocyanate composition or used after being mixed with other components described above. The polyisocyanate composition obtained in this manner and a polyol can be mixed to obtain a polyurethane resin forming composition according to one aspect of the present disclosure.

### <Polyurethane resin forming composition>

A polyurethane resin forming composition according to one aspect of the present disclosure includes the polyisocyanate composition according to the above-described embodiment and a polyol. The composition has a polyurethane resin-forming property, and a polyurethane resin is formed as a reaction product of a polyisocyanate and a polyol by reacting the polyisocyanate (for example, BDI derivative) with the polyol in the composition.

The polyol is a compound having two or more hydroxy groups, which are active hydrogen groups, as reactive groups with isocyanate groups. The polyol is not particularly limited, and polyester polyol, polyether polyol, polycarbonate polyol, polyolefin polyol, acrylic polyol, silicone polyol, castor oil-based polyol, fluorine-based polyol, transesterification products of two or more polyols, hydroxy group-terminated prepolymers subjected to a urethane-forming reaction with a polyisocyanate and the like are suitably used. The polyols may be used alone or two or more thereof may be used in combination.

### (Polyester polyol)

Examples of polyester polyols include those obtained by a condensation polymerization reaction between one or more dicarboxylic acids or their anhydrides and one or more low-molecular-weight polyols having a molecular weight of 500 or less. Examples of dicarboxylic acids include phthalic acid, isophthalic acid, terephthalic acid, naphthalene dicarboxylic acid, succinic acid, tartaric acid, oxalic acid, malonic acid, glutaric acid, adipic acid, pimelic acid, suberic acid, glutaconic acid, azelaic acid, sebacic acid, 1,4-cyclohexyldicarboxylic acid, α-hydromuconic acid, β-hydromuconic acid, α-butyl-α-ethylglutaric acid, α,β-diethylsuccinic acid, maleic acid, and fumaric acid. Examples of low-molecular-weight polyols having a molecular weight of 500 or less include ethylene glycol, 1,2-propanediol, 1,3-propanediol, 1,2-butanediol, 1,3-butanediol, 1,4-butanediol, 1,5-pentanediol, 1,6-hexanediol, 1,8-octanediol, 1,9-nonanediol, 3-methyl-1,5-pentanediol, 3,3-dimethylolheptane, diethylene glycol, dipropylene glycol, neopentyl glycol, cyclohexane-1,4-diol, cyclohexane-1,4-dimethanol, dimer diol, bisphenol A, ethylene oxide or propylene oxide adducts of bisphenol A, bis(β-hydroxyethyl)benzene, xylylene glycol, glycerin, trimethylolpropane, and pentaerythritol. In addition, lactone-based polyester polyols obtained by ring-opening polymerization of cyclic ester (so-called lactone) monomers such as ε-caprolactone, alkyl-substituted ε-caprolactone, δ-valerolactone, and alkyl-substituted δ-valerolactone can also be used. In addition, polyester-amide polyols obtained by replacing a part of a low-molecular-weight polyol with a low-molecular-weight polyamine such as hexamethylenediamine, isophoronediamine, and monoethanolamine or a low-molecular-weight amino alcohol can also be used.

### (Polyether polyol)

Examples of polyether polyols include polyether polyols obtained by addition polymerization of alkylene oxides using compounds having two or more, preferably two or three, active hydrogen groups such as low-molecular-weight polyols and low-molecular-weight polyamines as initiators. Examples of low-molecular-weight polyols include the same compounds as those exemplified as the low-molecular-weight polyols having a molecular weight of 500 or less. Examples of low-molecular-weight polyamines include ethylenediamine, propylenediamine, toluenediamine, metaphenylenediamine, diphenylmethanediamine, and xylylenediamine. Examples of alkylene oxides include ethylene oxide, propylene oxide, and butylene oxide. In addition, polyether polyols obtained by ring-opening polymerization of cyclic ether monomers, for example, alkyl glycidyl ethers such as methyl glycidyl ether, aryl glycidyl ethers such as phenyl glycidyl ether, and tetrahydrofuran can also be used.

### (Polycarbonate polyol)

Examples of polycarbonate polyols include those obtained by a dealcoholization reaction or a dephenolation reaction between one or more low-molecular-weight polyols and diaryl carbonates. Examples of low-molecular-weight polyols include the same compounds as those exemplified as the low-molecular-weight polyols having a molecular weight of 500 or less. Examples of diaryl carbonates include dialkyl carbonates such as dimethyl carbonate and diethyl carbonate, alkylene carbonates such as ethylene carbonate and propylene carbonate, diphenyl carbonate, dinaphthyl carbonate, dianthryl carbonate, diphenanthryl carbonate, diindanyl carbonate, and tetrahydronaphthyl carbonate. In addition, as the polycarbonate polyol, a polyol obtained by a transesterification reaction between a polycarbonate polyol, a polyester polyol and a low-molecular-weight polyol can also be used.

### (Polyolefin polyol)

Examples of polyolefin polyols include polybutadienes having two or more hydroxy groups, hydrogenated polybutadienes, polyisoprenes, and hydrogenated polyisoprenes.

### (Acrylic polyol)

Examples of acrylic polyols include those obtained from an acrylic acid ester and/or a methacrylic acid ester (hereinafter referred to as a (meth)acrylic acid ester), an acrylic acid hydroxy compound and/or a methacrylic acid hydroxy compound (hereinafter referred to as a (meth)acrylic acid hydroxy compound), and a polymerization initiator by polymerizing or copolymerizing the (meth)acrylic acid ester and/or (meth)acrylic acid hydroxy compound using light energy such as ultraviolet light or an electron beam or thermal energy.

### [(Meth)acrylic acid ester]

Examples of (meth)acrylic acid esters include alkyl esters having 1 to 20 carbon atoms. Examples of such (meth)acrylic acid esters include (meth)acrylic acid alkyl esters such as methyl (meth)acrylate, ethyl (meth)acrylate, propyl (meth)acrylate, butyl (meth)acrylate, pentyl (meth)acrylate, hexyl (meth)acrylate, 2-ethylhexyl (meth)acrylate, octyl (meth)acrylate, nonyl (meth)acrylate, decyl (meth)acrylate, and dodecyl (meth)acrylate; esters of (meth)acrylic acid such as cyclohexyl (meth)acrylate with alicyclic alcohols; and (meth)acrylic acid aryl esters such as phenyl (meth)acrylate and benzyl (meth)acrylate. The (meth)acrylic acid esters may be used alone or two or more thereof may be used in combination.

### [(Meth)acrylic acid hydroxy compound]

The (meth)acrylic acid hydroxy compound has at least one hydroxy group in its molecule that can serve as a reaction site with a polyisocyanate. Examples of (meth)acrylic acid hydroxy compounds include acrylic acid hydroxy compounds such as 2-hydroxyethyl acrylate, 2-hydroxypropyl acrylate, 4-hydroxybutyl acrylate, 3-hydroxy-2,2-dimethylpropyl acrylate, and pentaerythritol triacrylate. In addition, methacrylic acid hydroxy compounds such as 2-hydroxyethyl methacrylate, 2-hydroxypropyl methacrylate, 4-hydroxybutyl methacrylate, 3-hydroxy-2,2-dimethylpropyl methacrylate, and pentaerythritol trimethacrylate can also be used. The (meth)acrylic acid hydroxy compounds may be used alone or two or more thereof may be used in combination.

### (Silicone polyol)

Examples of silicone polyols include vinyl group-containing silicone compounds obtained by polymerizing γ-methacryloxypropyltrimethoxysilane and polysiloxanes having at least one terminal hydroxy group in its molecule such as α,ω-dihydroxypolydimethylsiloxane and α,ω-dihydroxypolydiphenylsiloxane.

### (Castor oil-based polyol)

Examples of castor oil-based polyols include linear or branched polyester polyols obtained by reacting castor oil fatty acids with polyols. In addition, a dehydrated castor oil, a partially dehydrated castor oil that is partially dehydrated, and a hydrogenated castor oil to which hydrogen is added can also be used.

### (Fluorine-based polyol)

Examples of fluorine-based polyols include linear or branched polyols obtained by a copolymerization reaction of a fluorine-containing monomer and a monomer having a hydroxy group as essential components. Here, the fluorine-containing monomer is preferably a fluoroolefin. Examples of fluorine-containing monomers include tetrafluoroethylene, chlorotrifluoroethylene, trichlorofluoroethylene, hexafluoropropylene, vinylidene fluoride, vinyl fluoride, and trifluoromethyltrifluoroethylene. In addition, examples of monomers having a hydroxy group include hydroxyalkyl vinyl ethers such as hydroxyethyl vinyl ether, 4-hydroxybutyl vinyl ether, and cyclohexane diol monovinyl ether, hydroxyalkyl allyl ethers such as 2-hydroxyethyl allyl ether, hydroxy group-containing vinyl carboxylates such as hydroxyalkyl vinyl crotonate, and hydroxy group-containing allyl esters.

The number of active hydrogen groups (average number of functional groups) in one polyol molecule is preferably 1.9 to 6.0. When the number of active hydrogen groups is within the above-described range, the obtained coating film tends to exhibit a better hardness.

The number-average molecular weight of the polyol is preferably in a range of 750 to 50,000. When the number-average molecular weight of the polyol is equal to or more than the lower limit value, the adhesion tends to be further improved. When the number-average molecular weight of the polyol is equal to or less than the upper limit value, the solubility in a low-polarity organic solvent is further improved, and the adhesion tends to be further improved.

In the polyurethane resin forming composition, the content ratio of the polyisocyanate composition and the polyol is not particularly limited, and the molar ratio R (isocyanate group/hydroxy group) of the isocyanate group in the polyisocyanate composition to the hydroxy group in the polyol is preferably 0.5 to 2.5. When the molar ratio R is equal to or more than the lower limit value, the number of hydroxy groups does not become excessive, and thus the adhesion tends to be further improved, a decrease in the crosslink density is reduced, and the durability and the mechanical strength of the coating film tend to be further improved. When the molar ratio R is equal to or less than the upper limit value, the number of isocyanate groups does not become excessive, the reaction with water in the air is inhibited, and thus swelling of the coating film is further reduced, and a decrease in the adhesion tends to be further reduced accordingly.

The polyurethane resin forming composition may be a one-liquid type composition in which all of the constituent components are contained in one liquid or a multi-liquid type composition in which constituent components are present separately in a plurality of liquids. The multi-liquid type composition may include, for example, a first liquid containing a polyol (main agent), and a second liquid formed of a polyisocyanate composition (curing agent).

The polyurethane resin forming composition may contain a diluting solvent. When the polyurethane resin forming composition is of a multi-liquid type, the diluting solvent may be contained in either of the first liquid and the second liquid or in both of them. The diluting solvent is, for example, an organic solvent. Examples of diluting solvents include ketones such as acetone, methyl ethyl ketone, and methyl isobutyl ketone, esters such as ethyl acetate, butyl acetate, and cellosolve acetate, alcohols such as butanol and isopropyl alcohol, and hydrocarbons such as toluene, xylene, cyclohexane, mineral spirit, and naphtha. The diluting solvent can be appropriately selected and used depending on the purpose and application. The diluting solvents may be used alone or two or more thereof may be used in combination.

The polyurethane resin forming composition may contain a urethane-forming catalyst. As the urethane-forming catalyst, a known urethane-forming catalyst can be appropriately used in consideration of a pot life, curing conditions, working conditions and the like. Examples of specific urethane-forming catalysts include organometallic compounds such as dibutyltin diacetate, dibutyltin dilaurate, and dioctyltin dilaurate, and organic amines such as triethylenediamine and triethylamine or salts thereof. The urethane-forming catalysts may be used alone or two or more thereof may be used in combination.

As necessary, for example, antioxidants such as 2,6-di-tert-butyl-4-methylphenol, and additives such as a UV absorbing agent, a pigment, a dye, a solvent, a flame retardant, a hydrolysis inhibitor, a lubricant, a plasticizer, a filler, an antistatic agent, a dispersant, a catalyst, a storage stabilizer, a surfactant, and a leveling agent can be appropriately added to the polyurethane resin forming composition. When the polyurethane resin forming composition is of a multi-liquid type, these additives may be contained in either of the first liquid and the second liquid or in both of them.

The polyurethane resin forming composition can be suitably used as a coating composition.

### <Coating composition and coating film>

The coating composition according to one aspect of the present disclosure includes the polyisocyanate composition according to the above-described embodiment, and a compound having two or more isocyanate-reactive groups (hereinafter referred to as an isocyanate-reactive compound). In addition, the coating film according to one aspect of the present disclosure includes a cured product of the above-described coating composition.

The isocyanate-reactive compound may be a polyol. That is, the coating composition may contain the polyurethane resin forming composition according to the above-described embodiment. The isocyanate-reactive compound may be any compound having two or more isocyanate-reactive groups (for example, active hydrogen groups) and may be a polyamine, an amino alcohol or the like.

The coating film has a high hardness because the coating composition contains the polyisocyanate composition according to the above-described embodiment. The Martens hardness of the coating film at 23°C is preferably 100 N/mm² or more, more preferably 104 N/mm² or more, and still more preferably 110 N/mm² or more. The Martens hardness can be measured according to JIS Z 2255.

The coating film can be formed by applying the coating composition onto the surface of an adherend by a known method such as spraying, brushing, immersing, or using a coater and curing it.

The adherend is not particularly limited, and adherends molded from materials such as stainless steel, phosphate-treated steel, galvanized steel, iron, copper, aluminum, brass, glass, slate, acrylic resins, polycarbonate resins, polyethylene terephthalate resins, polyethylene naphthalate resins, polybutylene phthalate resins, polystyrene resins, AS resins, ABS resins, polycarbonate-ABS resins, 6-nylon resins, 6,6-nylon resins, MXD6 nylon resins, polyvinyl chloride resins, polyvinyl alcohol resins, polyurethane resins, phenol resins, melamine resins, polyacetal resins, chlorinated polyolefin resins, polyolefin resins, polyamide resins, polyether ether ketone resins, polyphenylene sulfide resins, NBR resins, chloroprene resins, SBR resins, and SEBS resins, olefin resins such as polyethylene and polypropylene that have been subjected to a corona discharge treatment or other surface treatments, or an adherend having a coating film layer formed on the surface of the adherend, which can serve as an intermediate layer, and the like can be used.

Curing conditions for the coating composition are not particularly limited, and it is preferable that the curing temperature be -5 to 150°C, the humidity be 10 to 95% RH, and the curing time be 0.5 to 336 hours.

Because the coating film formed on the surface layer of the adherend provides excellent recoatability and durability, it is sufficient to form a coating film having a film thickness of at least 10 micrometers on the adherend. When the film thickness is 10 micrometers or more, the durability can be improved, and the occurrence of breakage of the coating film due to impact can be further reduced.

### [Examples]

Hereinafter, the contents of the present disclosure will be described in more detail with reference to example and comparative examples, but the present disclosure is not limited to the following examples.

### <Example 1>

### (Preparation of polyisocyanate composition)

800 g of BDI (butane diisocyanate, NCO content: 60.0 mass%) was put into a 1-L four-neck flask including a stirrer, a thermometer, a cooling tube, and a nitrogen gas inlet tube, and heated to 100°C, 9.6 g of hexamethyldisilazane as a isocyanurate-forming catalyst was added, the temperature was raised to 140°C, and the mixture was stirred for 2.6 hours. Then, the temperature was lowered to 50°C in a water bath, 6.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 179 g of a polyisocyanate composition P-1 formed of BDI derivatives. The properties of the obtained polyisocyanate composition P-1 are shown in Table 1. Here, in this example, the properties of the polyisocyanate composition were measured by the following method.

### (Measurement of molar ratio (I/U))

Based on a total content of 100 mol% of the isocyanurate group and the uretdione group in the BDI derivative, respective contents (unit: mol%) of the isocyanurate group and the uretdione group were determined by the ¹H-NMR method using ECX400M (product name, commercially available from JEOL Ltd.), and the molar ratio of the isocyanurate groups to the uretdione groups (molar ratio (I/U)) was determined from these contents.

Specifically, first, the polyisocyanate composition was dissolved in deuterated chloroform containing 0.2 mass% of tetramethylsilane to prepare a measurement sample. The sample concentration was 0.02 g/ 1mL mass%. For chemical shift reference, the signal of a hydrogen atom in tetramethylsilane was set to 0 ppm. The measurement sample was measured by ¹H-NMR, the signal areas of hydrogen atoms of methylene groups adjacent to nitrogen atoms of the isocyanurate ring and the uretdione ring were determined, and the contents of the isocyanurate group and the uretdione group were calculated by the following formula. Content of isocyanurate group (unit: mol%) = (signal area around 3.9 ppm / 6) / (signal area around 3.9 ppm / 6 + signal area around 3.3 ppm / 4) × 100 Content of uretdione group (unit: mol%) = (signal area around 3.3 ppm / 4) / (signal area around 3.9 ppm / 6 + signal area around 3.3 ppm / 4) × 100

### (Measurement of content of other BDI derivatives)

The content of compounds (other BDI derivatives) derived from BDI monomers other than the isocyanurate form and the uretdione form was determined by the ¹H-NMR method in the same manner as in the calculation of the molar ratio (I/U). Specifically, the content of other BDI derivatives was calculated by the following formula. Content of other BDI derivatives (unit: mol%) = (signal area around 6.5 to 8.5 ppm + signal area around 4.4 ppm to 5.4 ppm) / (signal area around 3.9 ppm / 6 + signal area around 3.3 ppm / 4 + signal area around 6.5 to 8.5 ppm + signal area around 4.4 ppm to 5.4 ppm) × 100

### (Measurement of content of BDI trimer and BDI dimer)

The sum of the contents of the BDI trimer and the BDI dimer (total content) was measured by GPC using the following device. Device: HLC-8420 (commercially available from Tosoh Corporation) Column: TSK gel (commercially available from Tosoh Corporation) One each of G3000H-XL, G2000H-XL, and G1000H-XL connected in series.
Carrier: tetrahydrofuran
Detection method: differential refractive index detector

The differential refractive index peak of the BDI trimer appeared at a holding time of about 23.8 minutes, and the differential refractive index peak of the BDI dimer appeared at a holding time of about 24.9 minutes. The areas of these peaks were determined and the total content of the BDI trimer and the BDI dimer was calculated by the following formula. Total content of the BDI trimer and the BDI dimer (mass%) = (peak area at a holding time of 23.8 minutes + peak area at a holding time of 24.9 minutes) / total area of all peaks × 100

### (Measurement of weight average molecular weight)

The weight average molecular weight of the obtained polyisocyanate composition (BDI derivative) was measured using the GPC device with polystyrene as the molecular weight standard.

### (Measurement of NCO content)

The NCO content of the polyisocyanate composition (BDI derivative) was determined by reacting the composition with a secondary amine and then back titrating the unreacted secondary amine with hydrochloric acid.

### (Measurement of viscosity)

The viscosity of the obtained polyisocyanate composition (BDI derivative) was measured in an environment at 25°C using a rheometer (HAAKE MARS60, commercially available from Thermo Fisher Scientific) .

### <Example 2>

800 g of BDI was put into a 1-L four-neck flask including a stirrer, a thermometer, a cooling tube, and a nitrogen gas inlet tube, and heated to 150°C, and the mixture was stirred for 4 hours. Then, 8.0 g of hexamethyldisilazane as an isocyanurate-forming catalyst was added, and the mixture was stirred for 3.6 hours. Then, the temperature was lowered to 50°C in a water bath, 5.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 227 g of a polyisocyanate composition P-2 formed of BDI derivatives. The properties of the obtained polyisocyanate composition P-2 are shown in Table 1.

### <Example 3>

800 g of BDI was put into a 1-L four-neck flask including a stirrer, a thermometer, a cooling tube, and a nitrogen gas inlet tube, and heated to 100°C, 8.0 g of hexamethyldisilazane as an isocyanurate-forming catalyst was added, the temperature was raised to 140°C, and the mixture was stirred for 3.0 hours. Then, the temperature was lowered to 50°C in a water bath, 5.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 184 g of a polyisocyanate composition P-3 formed of BDI derivatives. The properties of the obtained polyisocyanate composition P-3 are shown in Table 1.

### <Example 4>

800 g of BDI was put into a 1-L four-neck flask including a stirrer, a thermometer, a cooling tube, and a nitrogen gas inlet tube, and heated to 150°C, and the mixture was stirred for 3 hours. Then, 6.4 g of hexamethyldisilazane as an isocyanurate-forming catalyst was added, and the mixture was stirred for 2.0 hours. Then, the temperature was lowered to 50°C in a water bath, 4.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 161 g of a polyisocyanate composition P-4 formed of BDI derivatives. The properties of the obtained polyisocyanate composition P-4 are shown in Table 1.

### <Example 5>

800 g of BDI was put into a 1-L four-neck flask including a stirrer, a thermometer, a cooling tube, and a nitrogen gas inlet tube, and heated to 100°C, 3.2 g of hexamethyldisilazane as an isocyanurate-forming catalyst was added, the temperature was raised to 140°C, and the mixture was stirred for 4.0 hours. Then, the temperature was lowered to 50°C in a water bath, 2.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 112 g of a polyisocyanate composition P-5 formed of BDI derivatives. The properties of the obtained polyisocyanate composition P-5 are shown in Table 1.

### <Example 6>

800 g of BDI was put into a 1-L four-neck flask including a stirrer, a thermometer, a cooling tube, and a nitrogen gas inlet tube, and heated to 100°C, 1.6 g of hexamethyldisilazane as an isocyanurate-forming catalyst was added, the temperature was raised to 140°C, and the mixture was stirred for 5.0 hours. Then, the temperature was lowered to 50°C in a water bath, 1.0 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 88 g of a polyisocyanate composition P-6 formed of BDI derivatives. The properties of the obtained polyisocyanate composition P-6 are shown in Table 1.

### <Example 7>

800 g of BDI was put into a 1-L four-neck flask including a stirrer, a thermometer, a cooling tube, and a nitrogen gas inlet tube, and heated to 100°C, 0.8 g of hexamethyldisilazane as an isocyanurate-forming catalyst was added, the temperature was raised to 140°C, and the mixture was stirred for 6.0 hours. Then, the temperature was lowered to 50°C in a water bath, 0.5 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 83 g of a polyisocyanate composition P-7 formed of BDI derivatives. The properties of the obtained polyisocyanate composition P-7 are shown in Table 1.

### <Comparative Example 1>

800 g of BDI was put into a 1-L four-neck flask including a stirrer, a thermometer, a cooling tube, and a nitrogen gas inlet tube, and heated to 90°C, 11.2 g of N-trimethylsilyl-tertiary butylamine as an isocyanurate-forming catalyst was added, and the mixture was stirred for 3.5 hours. Then, the temperature was lowered to 50°C in a water bath, 3.9 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 84 g of a polyisocyanate composition P-8 formed of BDI derivatives. The properties of the obtained polyisocyanate composition P-8 are shown in Table 1.

### <Comparative Example 2>

800 g of BDI was put into a 1-L four-neck flask including a stirrer, a thermometer, a cooling tube, and a nitrogen gas inlet tube, and heated to 160°C, and the mixture was stirred for 1 hour. Then, 12.0 g of hexamethyldisilazane as an isocyanurate-forming catalyst was added, and the mixture was stirred for 5.6 hours. Then, the temperature was lowered to 50°C in a water bath, 7.5 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 339 g of a polyisocyanate composition P-9 formed of BDI derivatives. The properties of the obtained polyisocyanate composition P-9 are shown in Table 1.

### <Comparative Example 3>

800 g of BDI was put into a 1-L four-neck flask including a stirrer, a thermometer, a cooling tube, and a nitrogen gas inlet tube, and heated to 160°C, and the mixture was stirred for 3 hours. Then, the temperature was lowered to 90°C, 7.2 g of N-trimethylsilyl-diethylamine as an isocyanurate-forming catalyst was added, and the mixture was stirred for 4.3 hours. Then, the temperature was lowered to 50°C in a water bath, 2.5 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 139 g of a polyisocyanate composition P-10 formed of BDI derivatives. The properties of the obtained polyisocyanate composition P-10 are shown in Table 1. Since P-10 contained precipitates, the viscosity was not measured. In addition, for the same reason, since the compatibility with the main agent was poor, the Martens hardness and the curing start temperature were not evaluated.

### <Comparative Example 4>

800 g of BDI was put into a 1-L four-neck flask including a stirrer, a thermometer, a cooling tube, and a nitrogen gas inlet tube, and heated to 140°C, and the mixture was stirred for 3 hours. Then, 0.8 g of hexamethyldisilazane as an isocyanurate-forming catalyst was added, and the mixture was stirred for 2.0 hours. Then, the temperature was lowered to 50°C in a water bath, 0.5 g of ethanol as a reaction terminator was added, and the termination reaction was performed at 50°C for 0.5 hours. After the reaction solution was cooled, excess BDI was removed by thin-film distillation (conditions: 110°C, 0.04 kPa) to obtain 60 g of a polyisocyanate composition P-11 formed of BDI derivatives. The properties of the obtained polyisocyanate composition P-11 are shown in Table 1.

### <Comparative Example 5>

800 g of HDI (1,6-hexane diisocyanate, NCO content: 49.9 mass%) was put into a 1-L four-neck flask including a stirrer, a thermometer, a cooling tube, and a nitrogen gas inlet tube, and heated to 140°C, and the mixture was stirred for 2 hours. Then, 2.4 g of hexamethyldisilazane as an isocyanurate-forming catalyst was added, and the mixture was stirred for 3.0 hours. Then, the temperature was lowered to 50°C in a water bath, 1.5 g of ethanol as a reaction terminator was added, and the termination reaction was performed at 50°C for 0.5 hours. After the reaction solution was cooled, excess HDI was removed by thin-film distillation (conditions: 140°C, 0.04 kPa) to obtain 252 g of a polyisocyanate composition P-12 formed of HDI derivatives. The properties of the obtained polyisocyanate composition P-12 are shown in Table 1.

### <Comparative Examples 6 and 7>

In Comparative Example 6 and Comparative Example 7, C-HXLV (commercially available from Tosoh Corporation) produced from HDI (1,6-hexane diisocyanate) and Stabio D-370 (commercially available from Mitsui Chemicals Inc.) produced from PDI (1,5-pentane diisocyanate) were used respectively. The properties thereof are shown in Table 1.

### <Evaluation>

### (Evaluation of quick-drying property)

A coating composition (two-component coating composition) including the obtained polyisocyanate composition and an acrylic polyol was prepared, and the curing start temperature of the coating composition was measured by a rigid pendulum test using RPT-3000W (product name, commercially available from A&D Co., Ltd.) to evaluate the quick-drying property of the polyisocyanate composition. Specifically, first, an acrylic polyol (product name: ACRYDIC A-801, hydroxyl value: 50 mg KOH/resin g, solid content: 50 mass%, commercially available from DIC Corporation) and a polyisocyanate composition were mixed at an equivalent ratio of 1:1 of the hydroxy group and the isocyanate group, butyl acetate was then used to adjust the coating solid content to 50 mass%, and thereby a coating composition was obtained. Next, the coating composition was applied to a steel plate (JIS G 3141, product name: SPCC-SB, commercially available from Paltek Corporation) so that the thickness of the coating film was 20 µm. Next, the steel plate was heated at a temperature increase rate of 2.5°C/min, a temperature at which the period of a pendulum (frame: FRB-300, edge: RBE-160) began to decay was determined, and the temperature was defined as the curing start temperature of the coating film.

### (Evaluation of hardness of coating film)

A coating composition (two-component coating composition) including the obtained polyisocyanate composition and an acrylic polyol was prepared, a coating film (cured film) was formed using the coating composition, and the Martens hardness of the cured film was measured. Specifically, first, an acrylic polyol (product name: Dianal JR5689, hydroxyl value: 67 mg KOH/resin g, solid content: 61 mass%, commercially available from Mitsubishi Chemical Corporation) and a polyisocyanate composition were mixed at an equivalent ratio of 1:1 of the hydroxy group and the isocyanate group, butyl acetate was then used to adjust the coating solid content to 40 mass%, and thereby a coating composition was obtained. Next, the coating composition was applied to a steel plate (JIS G 3141, product name: SPCC-SB, treatment method: PF-1077, commercially available from Paltek Corporation) so that the thickness of the coating film (uncured film) was 20 µm. Then, the coating film was dried for 1 hour under an environment at a temperature of 23°C and a relative humidity of 50%, and the dried coating film was then heated in a dryer at 150°C for 30 minutes. Subsequently, the coating film after the heat treatment was cured under an environment at a temperature of 23°C and a relative humidity of 50% for 7 days or longer to obtain a coating film (cured film) including a cured product of the coating composition. The Martens hardness of the obtained coating film (cured film) was measured using HM2000 (product name, commercially available from Fischer Instruments K.K.) according to JIS Z 2255.

**[Table 1]**

| | Composition | Isocyanate type | Molar ratio I/U | Content of other derivatives (mol%) | Content of trimer (mass%) | Content of dimer (mass%) | Total content of trimer and dimer (mass%) | Weight average molecular weight (g/mol) | NCO content (mass%) | Viscosity (mPa.s) | Martens hardness (N/mm²) | Curing start temperature (°C) |
|---|---|---|---|---|---|---|---|---|---|---|---|---|
| Example 1 | P-1 | BDI | 79/21 | 15 | 60 | 11 | 71 | 643 | 26.1 | 2351 | 115 | 104 |
| Example 2 | P-2 | BDI | 71/29 | 13 | 42 | 15 | 57 | 682 | 25.4 | 2297 | 113 | 103 |
| Example 3 | P-3 | BDI | 75/25 | 12 | 49 | 13 | 62 | 625 | 26.2 | 1996 | 114 | 104 |
| Example 4 | P-4 | BDI | 57/43 | 10 | 44 | 23 | 67 | 574 | 26.6 | 639 | 110 | 104 |
| Example 5 | P-5 | BDI | 49/51 | 9 | 44 | 29 | 73 | 536 | 27.4 | 370 | 108 | 103 |
| Example 6 | P-6 | BDI | 33/67 | 6 | 36 | 42 | 78 | 494 | 28.2 | 148 | 104 | 103 |
| Example 7 | P-7 | BDI | 21/79 | 5 | 26 | 55 | 81 | 472 | 28.4 | 78 | 100 | 100 |
| Comparative Example 1 | P-8 | BDI | 98/2 | 20 | 58 | 4 | 62 | 603 | 26.1 | 4367 | 119 | 103 |
| Comparative Example 2 | P-9 | BDI | 84/16 | 15 | 36 | 8 | 44 | 788 | 25.1 | 3659 | 116 | 103 |
| Comparative Example 3 | P-10 | BDI | 44/56 | 19 | 37 | 25 | 62 | 799 | 26.8 | Solid precipitation | Not evaluated | Not evaluated |
| Comparative Example 4 | P-11 | BDI | 10/90 | 4 | 20 | 69 | 89 | 401 | 29.2 | 37 | 95 | 102 |
| Comparative | P-12 | HDI | 60/40 | 10 | 40 | 25 | 65 | 634 | 23.0 | 185 | 95 | 117 |
| Example 5 | | | | | | | | | | | | |
| Comparative Example 6 | P-13 | HDI | >99/<1 | 10 | 70 | 0 | 70 | 807 | 23.1 | 1187 | 105 | 118 |
| Comparative Example 7 | P-14 | PDI | >99/<1 | 7 | 60 | 0 | 60 | 834 | 24.6 | 2464 | 107 | 114 |

### (Evaluation results)

As shown in Table 1, the polyisocyanate compositions P-1 to P-7 had a low viscosity, and the coating films thereof had a favorable quick-drying property and coating film hardness. Particularly, the polyisocyanate compositions P-4 to P-6 achieved high levels of the viscosity, coating film hardness, and quick-drying property. The polyisocyanate compositions P-8 and P-9 had a high viscosity and poor workability. The polyisocyanate composition P-10 had poor compatibility with the main agent and it was difficult to produce a coating film using the polyisocyanate composition P-10. The polyisocyanate composition P-11 had a low viscosity and a favorable quick-drying property, but the hardness of the coating film was not sufficient. The polyisocyanate composition P-12 had a low viscosity, but was poor in both the quick-drying property and coating film hardness. The polyisocyanate compositions P-13 and P-14 had an insufficient quick-drying property. Accordingly, it can be said that the polyisocyanate compositions of Examples 1 to 7 had a low viscosity and an excellent workability and quick-drying property, and the hardness of the obtained coating film was excellent.

## Claims

1. A butane diisocyanate derivative having an isocyanurate group and a uretdione group,
wherein the molar ratio of the isocyanurate groups to the uretdione groups is 20/80 to 80/20, and
wherein the weight average molecular weight based on gel permeation chromatography is 790 g/mol or less.

2. The butane diisocyanate derivative according to claim 1, comprising
a butane diisocyanate trimer and a butane diisocyanate dimer,
wherein a total content of the butane diisocyanate trimer and the butane diisocyanate dimer based on a total mass of the butane diisocyanate derivatives is 45 to 95 mass%.

3. The butane diisocyanate derivative according to claim 1,
wherein the molar ratio of the isocyanurate groups to the uretdione groups is 50/50 or more.

4. The butane diisocyanate derivative according to claim 1,
wherein the molar ratio of the isocyanurate groups to the uretdione groups is 60/40 or less.

5. A method for producing the butane diisocyanate derivative according to any one of claims 1 to 4, comprising:
a first heating step of heating a reaction product comprising butane diisocyanate at 80°C or higher; and
a second heating step of heating the reaction product that has undergone the first heating step at 80 to 180°C in the presence of an isocyanurate-forming catalyst.

6. The method for producing a butane diisocyanate derivative according to claim 5,
wherein the isocyanurate-forming catalyst comprises an aminosilyl group-containing compound.

7. A polyisocyanate composition comprising the butane diisocyanate derivative according to any one of claims 1 to 4.

8. The polyisocyanate composition according to claim 7,
wherein the viscosity at 25°C is 2,500 mPa·s or less.

9. A polyurethane resin forming composition comprising the polyisocyanate composition according to claim 8 and a polyol.

10. A coating composition comprising the polyisocyanate composition according to claim 8 and a compound having two or more isocyanate-reactive groups.

11. A coating film comprising a cured product of the coating composition according to claim 10.
